## Europäisches Patentamt

## European Patent Office

## Office européen des brevets

(19)

(11) **EP 1 000 634 A1**

# (12) EUROPÄISCHE PATENTANMELDUNG

(43) Veröffentlichungstag:
**17.05.2000 Patentblatt 2000/20**

(51) Int Cl.⁷: $A61N\ 1/39$, $A61N\ 1/05$

(21) Anmeldenummer: **98811120.9**

(22) Anmeldetag: **10.11.1998**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(71) Anmelder: **Sulzer Osypka GmbH**
**79639 Grenzach-Wyhlen (DE)**

(72) Erfinder:
• **Geistert, Wolfgang, Dr.**
  **D-79618 Rheinfelden-Herten (DE)**

• **Moosdorf, Rainer, Prof.Dr.**
  **D-35041 Marburg (DE)**

(74) Vertreter: **Maucher, Wolfgang et al**
  **Schmitt, Maucher & Börjes,**
  **Patent- und Rechtsanwälte,**
  **Dreikönigstrasse 13**
  **79102 Freiburg (DE)**

## (54) Stimulationselektrode sowohl zur Defibrillation als auch zum Pacen

(57) Eine Stimulationselektrode (1) zum Pacen und/ oder zur Defibrillation des Herzes (H) weist im distalen Endbereich (2) mehrere einzelne Elektroden (3) auf, mit welchen elektrische Energie zum Pacen und/oder zur Defibrillation auf den Herzmuskel übertragen werden kann. Sowohl zur Defibrillation als auch zum Pacen sind die gleichen Elektroden (3;3a,3b,3c,3d,3e,3f) mit der jeweils erforderlichen elektrischen Energie beaufschlagbar.

Fig. 3

**Beschreibung**

Die Erfindung betrifft eine Stimulationselektrode zum Pacen (Schrittmachen) und/oder zum Defibrillieren des Herzes gemäss dem Oberbegriff des entsprechenden unabhängigen Patentanspruchs sowie eine Vorrichtung zum Pacen und/oder zum Defibrillieren des Herzes gemäss dem entsprechenden unabhängigen Patentanspruch.

Grundsätzlich werden mehrere Arten von Stimulation des Herzes mittels elektrischer Energie unterschieden. Zum Pacen ist eine niederenergetische Stimulation erforderlich, die hierfür erforderlichen Energien liegen typischerweise im Bereich von etwa 5 bis 1000 Mikrojoule. Pacen kommt bei bradykarden Herzrhythmusstörungen zur Anwendung. Im Unterschied hierzu ist zum Defibrillieren eine hochenergetische Stimulation erforderlich (ein Elektroschock), die hierfür erforderlichen Energien liegen typischerweise im Bereich von etwa 0.5 bis 10 Joule für eine Vorkammerdefibrillation bzw. im Bereich von bis zu 40 Joule für eine Hauptkammerdefibrillation. Eine Defibrillation kommt bei tachykarden Herzrhythmusstörungen - wie Kammerflimmern - zur Anwendung.

Bei der niederenergetischen Stimulation ("pacing") erfolgt die Energieabgabe lokal, d.h. es wird nur ein sehr kleines Gebiet des Myokards, also des Herzmuskels, erregt. Die Erregung breitet sich dann automatisch über den Herzmuskel aus. Im Unterschied dazu muss bei der hochenergetischen Schockabgabe ein grosser Teil des Myokards gleichzeitig stimuliert werden, die Energieabgabe erfolgt hier typischerweise linienförmig oder flächig, unter anderem auch, um das Gewebe zu schonen.

Eine Stimulationselektrode für die Defibrillation ist beispielsweise beschrieben in der EP-A-0,636,385. Dort wird mittels länglicher Elektroden, die jeweils in der Vorkammer befestigt werden, die für die Defibrillation erforderliche Energie zugeführt. Ferner sind in der linken Hauptkammer zwei Sensorelektroden vorgesehen, die auch zeitweise eine Schrittmacherfunktion übernehmen können. Es ist hier aber so, dass die Defibrillation mittels der länglichen Elektroden erfolgt, die in den Vorkammern befestigt sind, und die Schrittmacherfunktion allenfalls mittels der Sensorelektroden erfolgen kann. Für die verschiedenen Funktionen sind also gesonderte Elektroden vorgesehen, wobei jede Elektrode immer nur bei einer Funktion zum Einsatz kommt.

Eine weitere Stimulationselektrode für die Defibrillation ist beispielsweise in der EP-A-0,855,196 beschrieben. Auch dort ist bei einer Ausführungsvariante die Möglichkeit beschrieben, nicht nur eine Defibrillation sondern auch eine Schrittmacherfunktion bewirken zu können. Jedoch sind auch bei dieser Ausführungsvariante für die Defibrillation und für die Schrittmacherfunktion separate Elektroden vorgesehen, wobei die eine Elektrode nur für die Defibrillation, die andere Elektrode hingegen nur für die Schrittmacherfunktion eingesetzt wird.

Bei Patienten, die sowohl unter bradykarden als auch unter tachykarden Herzrhythmusstörungen leiden, müssen also nach den obigen Ausführungen sowohl Elektroden für die Schrittmacherfunktion als auch Elektroden für die Defibrillation am Herzen befestigt werden. Infolgedessen müssen dann auch mehrere Ausgänge nach ausserhalb des Herzbeutels und des Brustkorbs geschaffen werden.

Was die Schrittmacherfunktion betrifft, so ist noch bekannt, dass bestimmte Algorithmen die Wahrscheinlichkeit einer tachykarden Herzrhythmusstörung verringern können. Hierzu gehört das sogenannte "Multisite pacing", bei welchem durch mehrere Elektroden auf bzw. in den beiden Vorkammern das Myokard entweder gleichzeitig oder in einer bestimmten Sequenz erregt werden kann. Will man eine solche Therapie durchführen, so muss eine Vielzahl von Elektroden am Herzen befestigt werden.

Aufgabe der Erfindung ist es, eine Stimulationselektrode vorzuschlagen, welche sowohl zum Pacen als auch zum Defibrillieren des Herzes eingesetzt werden kann, welche auch eine für das Gewebe vergleichsweise schonende Übertragung der Energie für die Defibrillation ermöglicht, und bei welcher die Anzahl der am Herzen zu befestigenden Elektroden vergleichsweise gering ist. Weiterhin wünschenswert ist es, die Anzahl der Ausgänge aus dem Herzbeutel und aus dem Brustkorb zu minimieren.

Diese Aufgabe wird durch eine Stimulationselektrode gelöst, wie sie durch die Merkmale des unabhänigen Patentanspruchs charakterisiert ist. Besonders vorteilhafte Ausgestaltungen ergeben sich aus den Merkmalen der abhängigen Patentansprüche.

Insbesondere zeichnet sich die erfindungsgemässe Elektrode dadurch aus, dass sowohl zur Defibrillation als auch zum Pacen die gleichen Elektroden mit der jeweils erforderlichen elektrischen Energie beaufschlagbar sind. Dadurch kann einerseits die Anzahl der Elektroden vergleichsweise gering gehalten werden, andererseits ist durch die Tatsache, dass die erfindungsgemässe Stimulationselektrode mehrere einzelne Elektroden aufweist, eine vergleichsweise schonende Übertragung der elektrischen Energie, die für eine Defibrillation erforderlich ist, möglich.

Bei einem Ausführungsbeispiel der erfindungsgemässen Stimulationselektrode sind zur Defibrillation sämtliche Elektroden zugleich, zum Pacen jedoch lediglich einzelne davon, mit elektrischer Energie beaufschlagbar. Während die Beaufschlagung sämtlicher Elektroden mit jeweils einem Teil der zur Defibrillation erforderlichen Energie einerseits eine vergleichsweise schonende Übertragung der Energie auf das Gewebe ermöglicht, weil die gesamte zu übertragende Energie auf die grösstmögliche Anzahl Elektroden verteilt wird, ist es andererseits so, dass die für das Pacen erforderliche Energie, die ja wesentlich geringer ist und lokal übertragen werden sollte, nur mit Hilfe von einzelnen dieser Elektroden erfolgt. Mit Hilfe von einzelnen Elektroden soll heissen, dass durchaus auch mehrere Elektroden an der Übertragung der Energie beteiligt sein können, aber nicht sämtliche Elektroden zugleich.

Bei einem weiteren Ausführungsbeispiel können in einem proximal an den distalen Endbereich angrenzenden Bereich zumindest einige der zu den einzelnen Elektroden führenden Zuführdrähte mechanisch zu einer Gruppe von Zuführdrähten zusammengefasst sein. Eine derartige Aufteilung kann beispielsweise derart erfolgen, dass diejenigen Zuführdrähte, die zu Elektroden führen, die dafür vorgesehen sind ihre Energie auf den gleichen Bereich des Herzes zu übertragen, mechanisch zu einer Gruppe von Zuführdrähten zusammengefasst sind. Nach Bildung dieser Gruppen können sämtliche Gruppen mechanisch zu einem Bündel zusammengefasst sein.

Diese Zusammenfassung von Zuführdrähten zu Gruppen erleichtert die Handhabung der Stimulationselektrode, weil der Chirurg bei einer derartigen Anordnung sofort erkennt, welche Elektroden jeweils zu einer Gruppe gehören und beispielsweise in der rechten Vorkammer zu befestigen sind bzw. in jeweils anderen Bereichen des Herzens.

Die mechanische Zusammenfassung von Zuführdrähten zu Gruppen kann beispielsweise durch Verschweissung von Isolationen dieser Zuführdrähte erfolgen oder dass einzelne Zuführdrähte in einen gemeinsamen Schlauch geführt werden. Es können sogar einzelne Zuführdrähte elektrisch miteinander verbunden werden, aber nicht sämtliche, weil es sonst nicht mehr möglich wäre, nur einzelne Elektroden zum Pacen mit elektrischer Energie zu beaufschlagen, sondern immer nur alle Elektroden gemeinsam mit Energie beaufschlagt werden könnten. Die zu Gruppen zusammengefassten Zuführdrähte können dann gemeinsam mechanisch zu einem Bündel zusammengefasst sein, welches zum proximalen Endbereich weiterführt. Spätestens an der Stelle, an der die Zuführdrähte später den Brustkorb des Patienten durchdringen sollen, sind die Zuführdrähte bzw. die einzelnen Gruppen von Zuführdrähten vorteilhafterweise zu einem einzigen Bündel zusammengefasst.

Bei einem weiteren Ausführungsbeispiel können im proximalen Endbereich mehrere Zuführdrähte mechanisch und/oder elektrisch zusammengefasst sein. Im proximalen Endbereich können beispielsweise die Zuführdrähte, die bis vor Erreichen des proximalen Endbereichs in einem Schlauch als Bündel zusammengefasst sind, wieder aus diesem Bündel austreten, wobei aber auch im proximalen Bereich einzelne Zuführdrähte wieder mechanisch und/oder elektrisch zu Gruppen zusammengefasst sein können. So können beispielsweise Zuführdrähte, die zu Elektroden führen, die am gleichen Bereich des Herzens befestigt werden sollen, mechanisch zu einer Gruppe zusammengefasst sein, wobei wieder einzelne Zuführdrähte, jedoch nicht alle, auch elektrisch zusammengefasst sein können.

Bei einer Weiterbildung dieses Ausführungsbeispiels ist proximal derjenigen Stelle im proximalen Endbereich, an welcher die Zuführdrähte mechanisch und/oder elektrisch zusammengefasst sind, eine geringere Anzahl von Drähten weitergeführt als die Anzahl von Zuführdrähten, die distal zur Stelle ihrer mechanischen und/oder elektrischen Zusammenfassung im proximalen Endbereich vorhanden ist. Die nach ihrer mechanischen und/oder elektrischen Zusammenfassung noch weitergeführten Drähte sind in der Anzahl also geringer als vor ihrer Zusammenfassung, was den Vorteil mit sich bringt, dass weniger Drähte an der Brustnadel befestigt werden müssen. Die Brustnadel wird zum Durchstossen des Brustkorbs zum Herasführen der Zuführdrähte aus dem Brustkorb benötigt - ihr Durchmesser kann durch die beschriebene Massnahme jedoch geringer gehalten werden. Das elektrische Verbinden von Zuführdrähten vor dem Eintritt in die Brustnadel hat ausserdem den Vorteil, dass durch Abschneiden wahlweise distal oder proximal der Verbindungsstelle die einzelnen Zuführdrähte entweder verbunden bleiben oder separiert werden können. Dies ist vor allem nützlich für den Fall, dass nicht alle einzelnen Zuführdrähte auch einzeln benötigt werden. Durch die bestehen bleibenden Verbindungen reduziert sich die Anzahl zu befestigender Stecker und Anschlusshandlungen auf das medizinisch notwendige Mass.

Bei einem weiteren Ausführungsbeispiel ist zusätzlich eine separate Elektrode zur Überwachung der Ventrikelaktivität und ggf. zum Pacen für den Ventrikel vorgesehen. Diese Elektrode zur Überwachung kann insbesondere auch dazu benutzt werden, beim Auftreten von Herzrhythmusstörungen ein entsprechendes Signal an eine Einrichtung abzugeben, welche dann entscheidet, ob eine Energieabgabe zur Stimulation erfolgen soll oder nicht.

Ein weiterer Aspekt der Erfindung betrifft eine Vorrichtung zum Pacen und/oder zum Defibrillieren des Herzes mit einer Stimulationselektrode, wie sie in den vorstehenden genannten Ausführungsarten charakterisiert ist. Diese Vorrichtung umfasst noch eine Versorgungseinheit, welche die Elektroden ansteuert und sie abhängig vom Zustand des Herzes mit der jeweiligen für das Pacen bzw. für die Defibrillation benötigten elektrischen Energie versorgt.

Ein weiterer Aspekt der Erfindung betrifft ein Verfahren zum Betreiben einer Stimulationselektrode, welche im distalen Endbereich mehrere Elektroden aufweist, mit welchen elektrische Energie zum Pacen und/oder zum Defibrillieren auf den Herzmuskel übertragen werden kann. Sowohl beim Pacen als auch beim Defibrillieren werden die gleichen Elektroden mit elektrischer Energie beaufschlagt. In einer vorteilhaften Verfahrensvariante werden bei der Defibrillation sämtliche Elektroden zugleich, beim Pacen jedoch lediglich einzelne davon, mit elektrischer Energie beaufschlagt. Die Vorteile dieser Verfahren entsprechen den bereits weiter oben im Zusammenhang mit der Stimulationselektrode erläuterten Vorteilen.

Weitere vorteilhafte Ausgestaltungen sind aus den nachfolgend anhand der Zeichnung erläuterten Ausführungsbeispielen der erfindungsgemässen Stimulationselektrode ersichtlich. Es zeigen in schematischer Darstellung:

Fig. 1    ein Ausführungsbeispiel einer erfindungsgemässen Stimulationselektrode,

Fig. 2    die Einzelheit II aus Fig. 1 vergrössert

und

Fig. 3    ein Herz mit einem Ausführungsbeispiel einer Vorrichtung zum Pacen bzw. zur Defibrillation des Herzes, welche Vorrichtung eine erfindungsgemässe Stimulationelektrode umfasst.

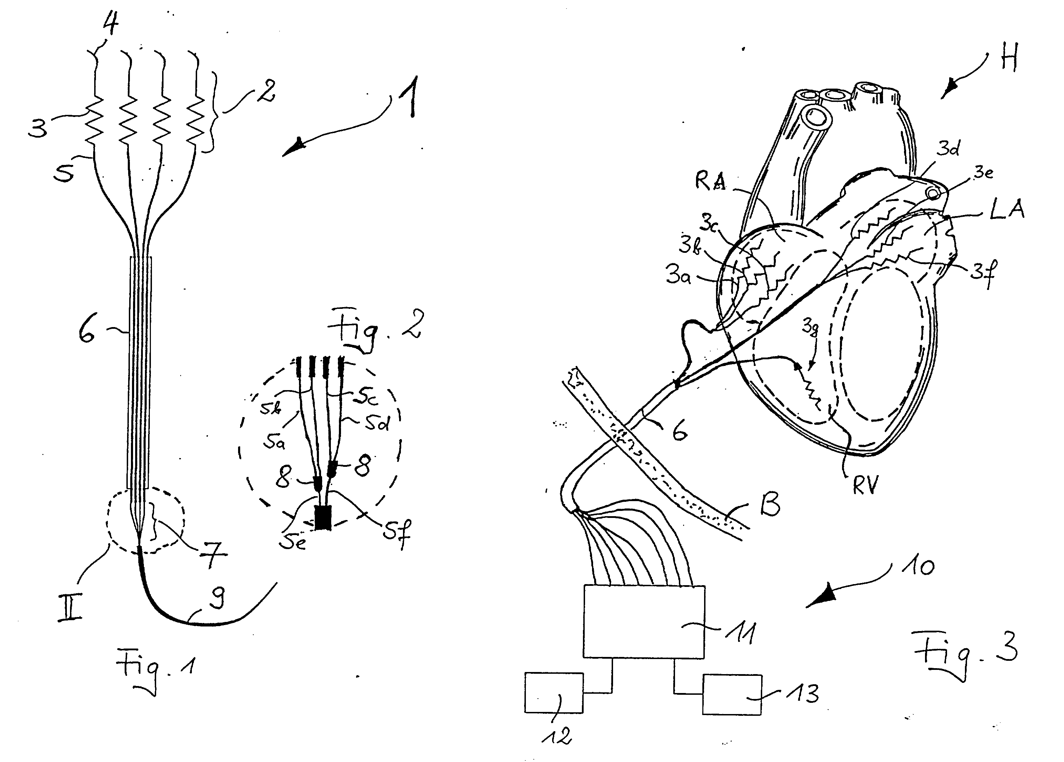

In dem in Fig. 1 gezeigten Ausführungsbeispiel einer erfindungsgemässen Stimulationselektrode 1 erkennt man im distalen Endbereich 2 mehrere Elektroden 3, die als abisolierte Drähte (z.B. Edelstahldraht bzw. Kupfer- oder Silberdraht, der von rostfreiem Stahl umgeben ist) oder Litzen ausgebildet sein können. Distal der Elektroden 3 sind Myokardnadeln 4 vorgesehen, mit deren Hilfe die Elektroden 3 in den Herzmuskel eingezogen werden können. Nach dem Einziehen der Elektroden 3 in den Herzmuskel können diese Myokardnadeln 4 abgeschnitten werden. Die einzelnen Elektroden 3 können durch ihre Gestalt lösbar am Herzmuskel befestigt sein, indem sie beispielsweise spiralförmig, zick-zack-förmig etc. ausgebildet sind oder mit kleinen Ankern (sog. "Tines") versehen sind. Vorzugsweise werden jedoch die einzelnen Elektroden 3 so augebildet, dass durch die Gestaltung der lösbaren Fixierung gleichzeitig die Berührungsfläche zwischen Elektrode und Herzmuskel vergrössert wird. Dafür bietet sich beispielsweise die in Fig. 1 gezeigte zick-zack-förmige Gestalt, aber beispielsweise auch eine wellenförmige Gestalt, der Elektroden 3 an.

Die maximale Grösse der Berührungsfläche $A_{Pace,max}$ einer Elektrode 3 mit dem Myokard richtet sich nach der akzeptablen Reizschwelle für das Pacen, und die Anzahl n der notwendigen Elektroden 3 richtet sich nach der für die Defibrillation notwendigen Berührungsfläche $A_{Defi,min}$ .
Es gilt:

$$n \geq A_{Defi,min} \, / \, A_{Pace,max}$$

Proximal anschliessend an den distalen Endbereich 2 sind Zuführdrähte 5 vorgesehen, welche zu den Elektroden 3 führen. Die Zuführdrähte 5 sind mit einer Ummantelung zur Isolation der Drähte bzw. Litzen versehen. Einige dieser Zuführdrähte 5 können - dies ist aber keineswegs ein Erfordernis - mechanisch zu Gruppen zusammengefasst sein (siehe auch Fig. 3). Beispielsweise können alle diejenigen Zuführdrähte 5, die zu Elektroden 3 führen, welche am selben Bereich des Herzens befestigt werden sollen, zu einer Gruppe mechanisch zusammengefasst werden (z.B. alle Zuführdrähte, die zu Elektroden führen, die in der rechten Vorkammer befestigt werden sollen). Das mechanische Zusammenfassen kann beispielsweise durch Verschweissen der Isolationsummantelungen der entsprechenden Zuführdrähte 5 erfolgen, ohne dass diese elektrisch miteinander verbunden sind. Es können auch einzelne Zuführdrähte und damit letzlich auch Elektroden elektrisch miteinander verbunden sein, jedoch nicht gleichzeitig alle miteinander.

Die einzelnen Zuführdrähte bzw. die einzelnen Gruppen von Zuführdrähten 5 sind vom distalen Enbereich 2 aus in proximaler Richtung betrachtet mechanisch zu einem Bündel zusammengefasst. Dieses Bündel kann so realisiert sein, dass die Zuführdrähte 5 bzw. Gruppen von Zuführdrähten 5 durch einen alle Zuführdrähte 5 bzw. alle Gruppen von Zuführdrähten 5 umhüllenden Schlauch 6 geführt sind. Im proximalen Endbereich 7 treten die Zuführdrähte 5 aus dem umhüllenden Schlauch 6 wieder aus.
Vorteilhafterweise sind die einzelnen Zuführdrähte 5 im proximalen Endbereich 7 so ausgebildet, dass sie voneinander unterschieden werden können. Dies kann beispielsweise durch eine entsprechende farbliche Gestaltung der Isolationsummantelungen erfolgen.

Auch im proximalen Endbereich 7 gilt, dass mehrere Zuführdrähte 5 wieder mechanisch zusammengefasst sein können. Einzelne Zuführdrähte 5 können auch elektrisch miteinander verbunden sein. Die Verbindung der Zuführdrähte 5 kann, je nachdem, ob die Verbindung rein mechanisch sein soll oder rein elektrisch oder beides, durch Krimphülsen, durch Verschweissen, durch Löten oder durch Kleben hergestellt werden. Man erkennt dies in der Einzelheit II in Fig. 2, wo die Zuführdrähte 5a und 5b wie auch die Zuführdrähte 5c und 5d an der Stelle 8 miteinander verbunden sind, und zwar sowohl mechanisch als auch elektrisch. In Fig. 1 und in Fig. 2 sind jeweils exemplarisch nur vier Zuführdrähte dargestellt, die Anzahl wurde jedoch nur aus Gründen der besseren Übersichtlichkeit so gewählt. Wie beispielsweise in Fig. 3 zu erkennen ist, kann die Anzahl der Zuführdrähte auch anders sein.

Nach der Stelle 8 (Fig. 2), an welcher die Zuführdrähte zusammengefasst sind, ist nur eine geringere Anzahl von Drähten - in Fig. 2 sind dies zwei Drähte 5e und 5f - weitergeführt als die Anzahl von Zuführdrähten vor der Stelle 8. Dies hat den Vorteil, dass eine geringere Anzahl von Drähten an der Brustnadel 9 befestigt werden muss. Dadurch kann der Durchmesser der Brustnadel 9 kleiner gehalten werden. Das Zusammenfassen von Zuführdrähten hat ausserdem den Vorteil, dass nach dem Durchführen der Brustnadel durch den Brustkorb durch Abschneiden der Brustnadel 9 wahlweise distal oder proximal der Stelle 8 die Zuführdrähte verbunden bleiben können oder wieder voneinander separiert werden können.

Im Falle von elektrisch miteinander verbundenen Zuführdrähten kann dies nützlich sein für den Fall, dass nicht alle Elektroden 3 auch tatsächlich einzeln ansteuerbar sein müssen. Sind einzelne Zuführdrähte sowohl elektrisch als auch mechanisch miteinander verbunden, so reduziert sich auch die Anzahl von zu befestigenden Steckern, die dann mit einer Versorgungseinrichtung verbunden werden können, und der entsprechenden Handlungen zum Anschluss dieser Stecker auf das medizinisch erforderliche Mass. Zur Vereinfachung beim Anschliessen solcher Stecker ist es von Vorteil, die Ummantelung der Zuführdrähte im proximalen Endbereich 7 ein kurzes Stück zu entfernen. Die abisolierten Enden können dann nach Befestigen eines Steckers mit den entsprechenden Anschlüssen einer Versorgungseinheit verbunden werden, sie können aber auch direkt mit der Versorgungseinheit verbunden werden.

Fig. 3 zeigt ein Ausführungsbeispiel einer Vorrichtung zum Pacen und/oder Defibrillieren des Herzens, wobei schematisch angedeutet ist, wie diese Vorrichtung bzw. deren Stimulationselektrode 1 mit dem Herzen H verbunden ist. Man erkennt, dass hier z.B. drei Elektroden 3a,3b,3c in der oder auf der rechten Vorkammer RA befestigt sind. Drei weitere Elektroden 3d, 3e,3f sind in der bzw. auf der linken Vorkammer LA befestigt. Die Elektroden 3a,3b,3c bzw. 3d,3e,3f sind jeweils für die Schrittmacherfunktion bzw. Defibrillation der Vorkammern RA bzw. LA vorgesehen sowie ferner für die Wahrnehmung der Vorkammeraktivitäten. Eine weitere bipolare Elektrode 3g (Sensorelektrode bzw. Schrittmacherfunktion) ist in der bzw. auf der Hauptkammer (hier der rechten Hauptkammer) RV befestigt, wobei die in diesem Ausführungsbeispiel gezeigte Anzahl der Elektroden nicht auf die hier gezeigte Anzahl von Elektroden beschränkt sein soll. Das Einziehen der Elektroden in das Myokard kann mit Hilfe der Myokardnadeln 4 (Fig. 1 ) am distalen Ende der Elektroden erfolgen, wobei die Myokardnadeln 4 nach erfolgtem Einziehen der Elektroden abgeschnitten werden. Die einzelnen Elektroden 3a,3b,3c bzw. 3d,3e,3f sowie 3g sind nach dem Einziehen so auf dem Myokard angeordnet, dass einerseits die für das Pacen und die Defibrillation notwendigen Herzmuskelbereiche berührt werden, andererseits ist die gesamte Stimulationselektrode 1 durch Herausziehen wieder entfernbar auf eine den Patienten schonende Weise. Die Anordnung der Elektroden ist daher vorzugsweise fächerförmig bis etwa parallel.

Man erkennt, dass die eizelnen Zuführdrähte, die jeweils zu dem gleichen Bereich des Herzens führen, jeweils mechanisch zu einer Gruppe zusammengefasst sind, was z.B. durch Verschweissen der einzelnen Ummantelungen erfolgen kann. Die so gebildeten einzelnen Gruppen von Zuführdrähten sind dann durch einen Schlauch 6 geführt, in welchem sie als Bündel zusammengefasst sind. Dieser Schlauch 6 wird durch den Brustkorb B des Patienten nach aussen geführt. Das Durchstossen des Brustkorbs B erfolgt mit Hilfe der Brustnadel 9 (Fig. 1), wobei nur ein einziges Bündel von Zuführdrähten nach aussen geführt werden muss, der Brustkorb B wird also nur an einer einzigen Stelle durchstossen. Ausserhalb des Brustkorbs B treten die Zuführdrähte wieder aus dem Schlauch 6 aus und können entweder einzeln, oder, wie bereits erläutert, mechanisch und/oder elektrisch zusammengefasst und mit einer Versorgungseinrichtung 10 (Fig. 3) verbunden werden.

Die in Fig. 3 schematisch dargestellte Versorgungseinrichtung 10 umfasst eine Umschalteinrichtung 11 sowie eine Schrittmachereinrichtung 12 und eine Defibrillationseinrichtung 13. Durch entsprechendes Verschalten der einzelnen Elektroden bzw. Zuführdrähte oder Gruppen solcher Zuführdrähte mit der Umschalteinrichtung 11 kann festgelegt werden, welche einzelnen Elektroden beim Pacen eingesetzt werden und welche Elektroden beim Defibrillieren zusammengeschaltet und eingesetzt werden. Beispielsweise können beim Pacen bei Bradykardie die Elektrode 3b als Kathode und die Elektroden 3a und 3c als Anode eingesetzt werden. Zum eingangs erwähnten "Multisite-Pacing", welches der Vorbeugung von Vorkammerflimmern dient, können in einer weiteren Schaltungsvariante beispielsweise die Elektroden 3a und 3b für die Stimulation der rechten Vorkammer RA und die Elektroden 3e und 3f für die Stimulation der linken Vorkammer LA eingesetzt werden. Wieder in einer weiteren Schaltungsvariante können zur Therapie von Vorkammerflimmern die Elektroden 3a,3b,3c und 3d,3e,3f zu Gruppen zusammengeschaltet werden und zwischen beiden Gruppen die Defibrillationsenergie abgegeben werden.

Die Umschaltung kann per Hand vorgenommen werden, allerdings ist auch eine etwas komplexere Ausgestaltung der Umschalteinrichtung 11 denkbar, bei welcher die Umschaltung je nach wahrgenommenem Zustand des Herzens automatisch erfolgt. Als Umschaltkriterien können hierzu beispielsweise die Herzfrequenz, die Änderung der Herzfrequenz oder die EKG-Kurvenform dienen. Selbstverständlich kann die Umschalteinrichtung 11 auch in die Schrittmachereinrichtung 12 bzw. in die Defibrillationseinrichtung 13 integriert sein, bzw. alle drei Einrichtung können eine einzige Einheit bilden.

Mit der erfindungsgemässen Stimulationselektrode ist es möglich, einerseits die Schrittmacherfunktion auszuüben und das Myokard lokal mit den hierfür üblichen Energien zu stimulieren, andererseits ist es möglich, durch Zusammenschaltung von mehreren oder auch allen einzelnen Elektroden die für die Defibrillation erforderliche Energie an eine genügend grosse Myokardmasse abzugeben, sodass das Herz defibrilliert wird, jedoch das Gewebe nicht lokal geschädigt wird. Die vorstehende Beschreibung bezieht sich zwar im wesentlichen auf die Vorkammerstimulation, die Anordnungen sind aber sinngemäss auch auf eine Hauptkammerstimulation übertragbar.

**Patentansprüche**

1. Stimulationselektrode (1) zum Pacen und/oder zum Defibrillieren des Herzes (H), welche im distalen Endbereich (2) mehrere einzelne Elektroden (3) aufweist, mit welchen elektrische Energie zum Pacen und/oder zur Defibrillation auf den Herzmuskel übertragen werden kann, dadurch gekennzeichnet, dass sowohl zur Defibrillation als auch zum Pacen die gleichen Elektroden (3;3a,3b,3c,3d,3e,3f) mit der jeweils erforderlichen elektrischen Energie beaufschlagbar sind.

2. Stimulationselektrode nach Anspruch 1, dadurch gekennzeichnet, dass zur Defibrillation sämtliche Elektroden (3;3a,3b,3c,3d,3e,3f) zugleich, zum Pacen jedoch lediglich einzelne (3a,3b,3c) davon, mit elektrischer Energie beaufschlagbar sind.

3. Stimulationselektrode nach Anspruch 1 oder 2, dadurch gekennzeichnet, dass die zum Pacen mit Energie beaufschlagbaren Elektroden aus der Gesamtheit der Elektroden auswählbar sind.

4. Stimulationselektrode nach einem der vorangehenden Ansprüche, dadurch gekennzeichnet, dass in einem proximal an den distalen Endbereich (2) angrenzenden Bereich zumindest einige der zu den einzelnen Elektroden führenden Zuführdrähte (5) mechanisch zu einer Gruppe von Zuführdrähten (5) zusammengefasst sind.

5. Stimulationselektrode nach Anspruch 4, dadurch gekennzeichnet, dass diejenigen Zuführdrähte (5), die zu Elektroden (3) führen, die dafür vorgesehen sind ihre Energie auf den gleichen Bereich (RA,LA) des Herzes (H) zu übertragen, mechanisch zu einer Gruppe von Zuführdrähten (5) zusammengefasst sind, und dass nach Bildung dieser Gruppen sämtliche Gruppen mechanisch zu einem Bündel (6) zusammengefasst sind.

6. Stimulationselektrode nach einem der vorangehenden Ansprüche, dadurch gekennzeichnet, dass im proximalen Endbereich (7) mehrere Zuführdrähte (5a,5b;5c,5d) mechanisch und/oder elektrisch zusammengefasst sind.

7. Stimulationselektrode nach Anspruch 6, dadurch gekennzeichnet, dass proximal derjenigen Stelle (8) im proximalen Endbereich (7), an welcher die Zuführdrähte (5a,5b;5c,5d) mechanisch und/oder elektrisch zusammengefasst sind, eine geringere Anzahl von Drähten (5e,5f) weitergeführt ist als die Anzahl von Zuführdrähten, die distal zur Stelle (8) ihrer mechanischen und/oder elektrischen Zusammenfassung im proximalen Endbereich (7) vorhanden ist.

8. Stimulationselektrode nach einem der vorangehenden Ansprüche, dadurch gekennzeichnet, dass zusätzlich eine separate Elektrode (3g) zur Überwachung der Ventrikelaktivität und ggf. zum Pacen für den Ventrikel (RV) vorgesehen ist.

9. Vorrichtung zum Pacen und/oder zum Defibrillieren des Herzes, mit einer Stimulationselektrode (1) nach einem der vorangehenden Ansprüche und mit einer Versorgungseinheit (10,11,12,13), welche die Elektroden (3;3a,3b,3c,3d,3e,3f,3g) ansteuert und sie abhängig vom Zustand des Herzes (H) mit der jeweiligen für das Pacen bzw. für die Defibrillation benötigten elektrischen Energie versorgt.

10. Verfahren zum Betreiben einer Stimulationselektrode, welche im distalen Endbereich (2) mehrere Elektroden (3) aufweist, mit welchen elektrische Energie zum Pacen und/oder zum Defibrillieren auf den Herzmuskel übertragen werden kann, dadurch gekennzeichnet, dass sowohl beim Pacen als auch beim Defibrillieren die gleichen Elektroden (3;3a, 3b,3c,3d,3e,3f) mit elektrischer Energie beaufschlagt werden.

11. Verfahren nach Anspruch 10, dadurch gekennzeichnet, dass bei der Defibrillation sämtliche Elektroden (3;3a,3b,3c,3d,3e,3f) zugleich, beim Pacen jedoch lediglich einzelne (3a,3b,3c) davon, mit elektrischer Energie beaufschlagt werden.

Fig. 1

Fig. 2

Fig. 3

EP 1 000 634 A1

**Europäisches Patentamt**

## EUROPÄISCHER TEILRECHERCHENBERICHT

der nach Regel 45 des Europäischen Patentübereinkommens für das weitere Verfahren als
europäischer Recherchenbericht gilt

**Nummer der Anmeldung**

EP 98 81 1120

### EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.6) |
|---|---|---|---|
| X | WO 98 47564 A (EP TECHNOLOGIES) 29. Oktober 1998 * Seite 8, Zeile 9-25 * * Seite 12, Zeile 17 - Seite 14, Zeile 28 * * Seite 27, Zeile 17 - Seite 29, Zeile 28 * * Seite 33, Zeile 10 - Seite 34, Zeile 29; Abbildungen 1-14 * | 1-9 | A61N1/39 A61N1/05 |
| X | EP 0 211 166 A (OSYPKA PETER) 25. Februar 1987 * Seite 9, Zeile 13 - Seite 10, Zeile 20 * * Seite 12, Zeile 17-20 * * Seite 13, Zeile 24-36; Abbildungen * | 1 | |
| Y | | 2-6,9 | |
| X | EP 0 799 628 A (INCONTROL INC) 8. Oktober 1997 * Spalte 4, Zeile 4-53 * * Spalte 6, Zeile 37 - Spalte 9, Zeile 54; Abbildungen * | 1 | |
| Y | | 2-6,9 | RECHERCHIERTE SACHGEBIETE (Int.Cl.6) |
| | | | A61N |

-/--

### UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche·

1-9

Unvollständig recherchierte Patentansprüche.

Nicht recherchierte Patentansprüche·

10-11

Grund für die Beschränkung der Recherche

Artikel 52 (4) EPÜ - Verfahren zur therapeutischen Behandlung des menschlichen oder tierischen Körpers

| Recherchenort | Abschlußdatum der Recherche | Prüfer |
|---|---|---|
| DEN HAAG | 14. April 1999 | Allen, E |

KATEGORIE DER GENANNTEN DOKUMENTEN

X · von besonderer Bedeutung allein betrachtet
Y . von besonderer Bedeutung in Verbindung mit einer anderen Veröffentlichung derselben Kategorie
A : technologischer Hintergrund
O : nichtschriftliche Offenbarung
P · Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

**Europäisches**
**Patentamt**

**Nummer der Anmeldung**

EP 98 81 1120

**EUROPÄISCHER**
**TEILRECHERCHENBERICHT**

| | EINSCHLÄGIGE DOKUMENTE | | KLASSIFIKATION DER ANMELDUNG (Int.Cl.6) |
|---|---|---|---|
| Kategorie | Kennzeichnung des Dokuments mit Angabe, soweit erforderlich der maßgeblichen Teile | Betrifft Anspruch | |
| X | US 5 181 511 A (NICKOLLS PETER ET AL) 26. Januar 1993 * Spalte 1, Zeile 17-21 * * Spalte 6, Zeile 59 – Spalte 7, Zeile 34 * * Spalte 9, Zeile 27 – Spalte 11, Zeile 68; Abbildungen * | 1-4 | |
| X | US 5 683 429 A (MEHRA RAHUL) 4. November 1997 * Spalte 7, Zeile 17 – Spalte 10, Zeile 25; Abbildungen 1-7 * | 1-3,9 | |
| X,D | EP 0 855 196 A (SULZER OSYPKA GMBH) 29. Juli 1998 * Spalte 4, Zeile 17-56; Abbildungen * | 1 | **RECHERCHIERTE SACHGEBIETE** (Int.Cl.6) |
| A | | 4-6 | |

EPO FORM 1503 03.82 (P04C12)

**ANHANG ZUM EUROPÄISCHEN RECHERCHENBERICHT
ÜBER DIE EUROPÄISCHE PATENTANMELDUNG NR.**

EP 98 81 1120

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-04-1999

| Im Recherchenbericht angeführtes Patentdokument | Datum der Veröffentlichung | Mitglied(er) der Patentfamilie | Datum der Veröffentlichung |
|---|---|---|---|
| WO 9847564 A | 29-10-1998 | US 5855592 A<br>AU 7153798 A | 05-01-1999<br>13-11-1998 |
| EP 0211166 A | 25-02-1987 | DE 3523226 A<br>AT 49127 T | 08-01-1987<br>15-01-1990 |
| EP 0799628 A | 08-10-1997 | US 5766224 A<br>CA 2200288 A<br>DE 799628 T | 16-06-1998<br>02-10-1997<br>05-03-1998 |
| US 5181511 A | 26-01-1993 | EP 0538990 A | 28-04-1993 |
| US 5683429 A | 04-11-1997 | AU 1971797 A<br>WO 9740886 A | 19-11-1997<br>06-11-1997 |
| EP 0855196 A | 29-07-1998 | JP 10211289 A | 11-08-1998 |

EPO FORM P0461

Für nähere Einzelheiten zu diesem Anhang : siehe Amtsblatt des Europäischen Patentamts, Nr. 12/82